# EUROPEAN PATENT SPECIFICATION

(11) **EP 0 600 866 B1**
(45) Date of publication and mention of the grant of the patent: **03.12.1997**
(21) Application number: 91910915.7
(22) Date of filing: 13.05.1991
(51) Int. Cl.: C12N 15/10, C12N 15/62, C12N 15/34, C12N 15/70, C12N 15/00, C07K 7/06

(54) **COMPOSITIONS AND METHODS FOR IDENTIFYING BIOLOGICALLY ACTIVE MOLECULES**
ZUSAMMENSETZUNGEN UND VERFAHREN ZUR IDENTIFIZIERUNG VON MOLEKÜLEN MIT BIOLOGISCHER WIRKSAMKEIT
COMPOSITIONS ET PROCEDES D'IDENTIFICATION DE MOLECULES BIOLOGIQUEMENT ACTIVES

(30) Priority: 01.06.1990 US 533180
(43) Date of publication of application: 15.06.1994
(73) Proprietor: CHIRON CORPORATION, Emeryville, California 94608-2916 (US)
(72) Inventor: DEVLIN, James, J., Lafayette, CA 94549 (US)
(74) Representative: Hallybone, Huw George
(86) International application number: US9103332
(87) International publication number: WO9118980

(56) References cited:
- US-A- 4 713 366
- SCIENCE, vol. 249, 27 July 1990, Lancaster, PA (US); J.J. DEVLIN et al., pp. 404-406
- GENE, vol. 73, 1988, Elsevier Science Publishers, B.V., Amsterdam (NL); S.F. PARMLEY et al., pp. 305-318
- JOURNAL OF BIOLOGICAL CHEMISTRY, vol. 264, no. 34, 05 December 1989, American Society for Biochemistry & Molecular Biology Inc., Baltimore, MD (US); B.D. LEMIRE et al., pp. 20206-20215
- PROTEIN ENGINEERING, vol. 2, no. 4, 1988, Eynsham (GB); I.S. DUNN et al., pp. 283-291
- JOURNAL OF HISTOCHEMISTRY & CYTO-CHEMISTRY, vol. 34, no. 11, 1986, Histochemical Society Inc., New York, NY (US); L. SCOPSI et al., pp. 1469-1475
- SCIENCE, vol. 24, 1988, Lancaster, PA (US); J.F. REIDHAAR-OLSON et al., pp. 53-57

## Description

This invention is in the area of molecular biology and presents compositions and methods for identifying biologically active molecules that will be used as medicaments in the clinical setting. The invention also has applications in the fields of clinical immunology and pharmacology.

A primary goal of molecular biology is to identify biologically active molecules that have practical clinical utility. The general approach taken by molecular biologists has been to initially identify a biological activity of interest, and then purify the activity to homogeneity. Next, assuming the molecule is a protein, the protein is sequenced and the sequence information used to generate synthetic DNA oligonucleotides that represent potential codon combinations that encode the protein of interest. The oligonucleotide is then used as a probe to probe a cDNA library derived from messenger RNA that was in turn derived from a biological source that produced the protein. The cDNA sequence so identified may be manipulated and expressed in a suitable expression system.

A second, more recent approach, termed expression cloning, avoids purifying and sequencing the protein of interest, as well as generating oligonucleotide probes to screen a cDNA library. Rather this procedure consists of initially ascertaining the presence of a biologically active molecule, generating cDNA from messenger RNA and directly cloning the cDNA into a suitable expression vector. The vector is typically an expression plasmid that is transfected or micro-injected into a suitable host cell to realize expression of the protein. Pools of the plasmid are assayed for bioactivity, and by narrowing the size of the pool that exhibits activity, ultimately a single clone that expresses the protein of interest is isolated.

Aside from the above approaches, it is, of course, well known that bioactive molecules other than proteins are constantly being isolated and screened in large numbers using traditional screening regimens well known to those that work in this field. Additionally, after a drug is identified and its chemical structure elucidated, attempts are made to synthesize more active versions of the drug by rational drug design.

Previously, it was suggested than an "epitope library" might be made by cloning synthetic DNA that encodes random peptides into filamentous phage vectors. Parmley and Smith, 1988, Gene, 73:305. It was proposed that the synthetic DNA be cloned into the coat protein gene III because of the likelihood of the encoded peptide becoming part of pIII without significantly interfering with pIII's function. It is known that the amino terminal half of pIII binds to the F pilus during infection of the phage into E. coli. It was suggested that such phage that carry and express random peptides on their cell surface as part of pIII may provide a way of identifying the epitopes recognized by antibodies, particularly using antibody to affect the purification of phage from the library. Parmley and Smith, 1988, Gene, 73:305. Unfortunately, this approach to date has not produce any useful biologically active molecules.

Previous investigators have shown that the outer membrane protein, LamB, of E. coli can be altered by genetic insertion to produce hybrid proteins having inserts up to about 60 amino acid residues. Charbit, A., Molla A., Saurin, W., and Hofnung, 1988, Gene. 70:181. The authors suggests that such constructs may be used to produce live bacterial vaccines. See also, Charbit, A., Boulain, J. C. Ryter, A. and Hofnung, M., 1986, EMBO J., 5(11):3029; and, Charbit, A., Sobezak, E., Michael, M.L., Molla, A., Tiollais, P., and Hofnung, M., 1987, J. Immunol., 139(5):1658.

The procedures that are presently used to identify protein bioactive molecules, as well as small molecular weight molecules, require a significant commitment of resources which often limit the progress of such projects. Thus, other methods that facilitate the identification of bioactive molecules are keenly sought after, and would have wide applicability in identifying medicaments of significant practical utility.

According to the present invention, there is provided a random peptide library comprising a plurality of fusion proteins, each fusion protein comprising a viral surface protein and a random peptide sequence of between six and sixteen amino acids at the amino terminal region of the viral surface protein wherein each fusion protein has a linker of about six amino acids between the C-terminus of the said peptide sequence and the viral surface protein.

One aspect of the invention is the description of a method for constructing a library of random peptides that may be used to identify bioactive peptides.

A second aspect of the invention is the description of a method and compositions for generating a random peptide library by cloning synthetic DNA, which encodes the random peptide sequences, into an appropriate expression vector.

A third aspect of the invention is the description of a random peptide library wherein the peptides are expressed on the surface of infectious filamentous phage that permits screening greater than 106 random peptide sequences at a time.

A fourth aspect of the invention is the identification of biotin inhibitable streptavidin binding peptides using a random peptide library of about 2 x 10⁷ different fifteen residue peptides. The peptides have a dipeptide amino acid consensus sequence of His-Pro, and may have a tripeptide amino acid consensus sequence of His-Pro-Gln.

A fifth aspect of the invention is a description of a defined filamentous phage expression system having random peptides expressed as a fusion protein with an appropriate phage protein such that the random peptides do not substantially interfere with the normal biological activities of the phage.

A sixth aspect of the invention is a description of a defined filamentous phage expression system having random peptides expressed as a fusion protein with an appropriate phage protein, the phage protein being expressed on the surface of the phage such that the random peptides are thus exposed and readily available for screening to determine their biological properties.

A seventh aspect of the invention is a description of a preferred filamentous phage expression system having random peptides expressed as a fusion protein with an appropriate phage protein, the phage protein being expressed on the surface of the phage, and the random peptides positioned at the amino terminal region of the phage protein. Positioning the random peptides at the amino terminal region facilitates screening the peptides for biological activity.

An eighth aspect of the invention is a description of a preferred filamentous phage expression system having random peptides expressed as a fusion protein with an appropriate phage protein, the phage protein being expressed on the surface of the phage, and the random peptides positioned at the amino terminal region of the phage protein, thereby facilitating screening the peptides for biological activity, wherein the fusion protein has the following construct:

V-----RP-----L----V₁

V stands for one or more amino acids of the wild type phage protein found at the amino terminus; RP stands for the random peptide sequence; L stands for a spacer sequence that facilitates presentation of the random peptide sequence to screening reagents; and V₁ stands for the amino acid sequence of the phage protein.

In addition to the foregoing, other aspects of the invention will become apparent upon reading the detailed description of the invention presented below.

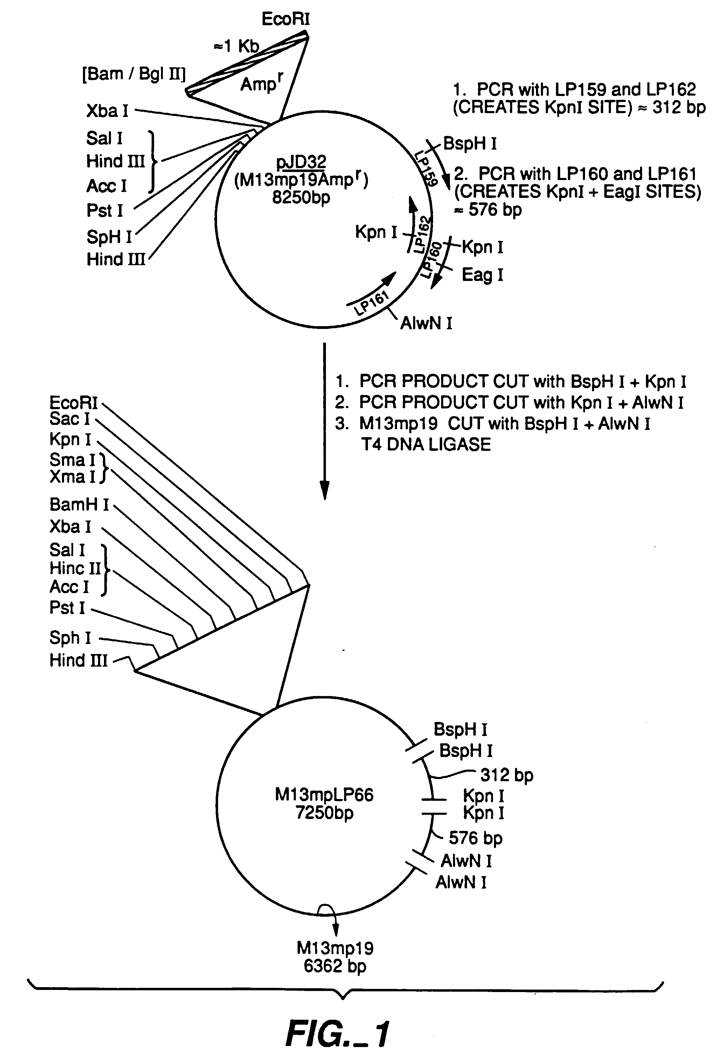

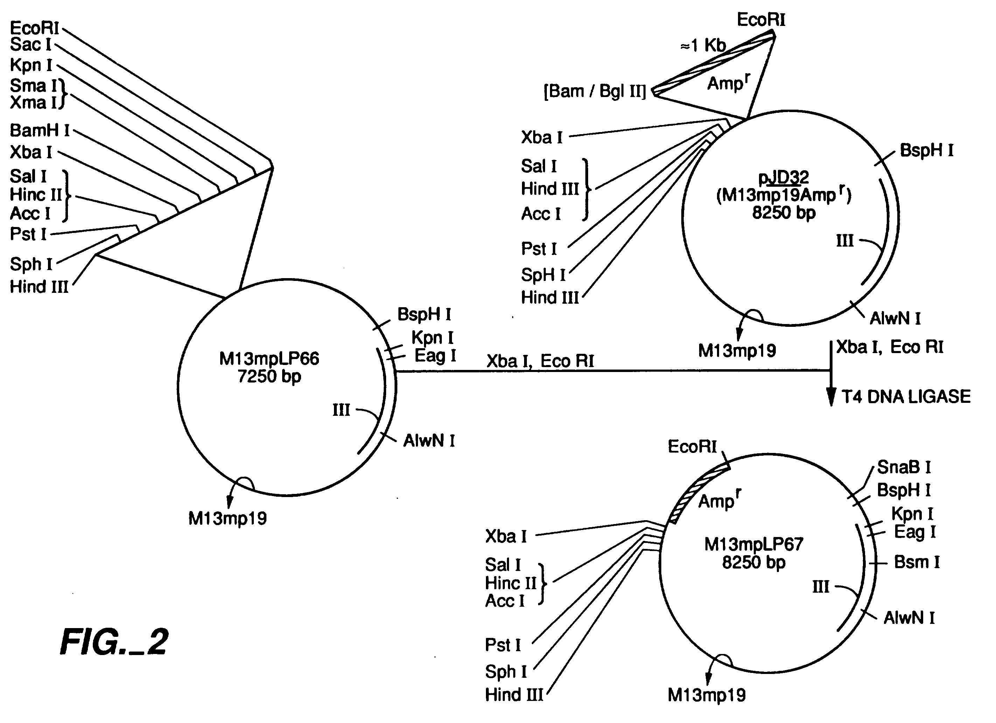

Figures 1 and 2 show the construction of M13LP67.

Table 1 shows the results of enriching for M13LP67 virions that encode streptavidin binding peptides that are present on the surface of the virions. The Table also shown that biotin inhibits the binding of certain of the peptides to streptavidin.

Table 2 presents the predicted amino acid sequence of the random peptides. encoded by several streptavidin binding phage.

The invention described herein draws on previously published work and pending patent applications. By way of example, such work consists of scientific papers, patents or pending patent applications. All of these publications and applications, cited previously or below are hereby incorporated by reference.

Described herein is a method for producing a library consisting of random peptide sequences. The library can be used for many purposes including identifying and selecting peptides that have a particular bioactivity, as well using such peptides, in those instances where they have ligand properties, to isolate and purify ligand binding molecules. An example of a ligand binding molecule would be a soluble or insoluble cellular receptor (i.e. membrane bound receptor), but would extend to virtually any molecule, including enzymes, that have the sought after binding activity.

"Cells" or "recombinant host" or "host cells" are often used interchangeably as will be clear from the context. These terms include the immediate subject cell, and, of course, the progeny thereof. It is understood that not all progeny are exactly identical to the parental cell, due to chance mutations or differences in environment.

As used herein the term "transformed" in describing host cell cultures denotes a cell that has been genetically engineered to produce a heterologous protein that possesses the activity of the native protein. Examples of transformed cells are described in the examples of this application. Bacteria are preferred microorganisms for producing the protein. Synthetic protein may also be made by suitable transformed yeast and mammalian host cells.

"Operably linked" refers to juxtaposition such that the normal function of the components can be performed. Thus, a coding sequence "operably linked" to control sequences refers to a configuration wherein the coding sequence can be expressed under the control of these sequences.

"Control sequences" refers to DNA sequences necessary for the expression of an operably linked coding sequence in a particular host organism. The control sequences which are suitable for procaryotes, for example, include a promoter, optionally an operator sequence, a ribosome binding site, and possibly, other as yet poorly understood, sequences. Eucaryotic cells are known to utilize promoters, polyadenylation signals, and enhancers.

"Expression system" refers to DNA sequences containing a desired coding sequence and control sequences in operable linkage, so that hosts transformed with these sequences are capable of producing the encoded proteins. In order to effect transformation, the expression system may be included on a vector; however, the relevant DNA may then also be integrated into the host chromosome.

The term "mature" protein is known in the art, and is intended to denote proteins that have a peptide segment of the protein removed during in vivo, or in vitro synthesis.

One embodiment of the invention is the description of methods and compositions for constructing a library of random peptides consisting of cloning synthetic DNA, which has a degenerate coding sequence that encodes the random peptide sequences, into an appropriate expression vector that has control sequences in operable linkage for regulation and expression of the random peptide sequence. The vector is then inserted into an appropriate host cell compatible with expression of the random peptide sequence. For example, depending on the nature of the vector, it may be transfected, transformed, electroporated, or in other ways inserted into the host cell. The random peptide sequence may be expressed as part of a fusion protein construct. Subsequently, cells that harbor the random peptide sequences are selected, isolated, and grown up. The random peptide sequence may be expressed in soluble or insoluble form (i.e. membrane bound), and, if desired, purified prior to being employed in biological screens aimed at identifying its properties.

The preferred embodiment synthetic DNA degenerate coding sequence that encodes the random peptide is exemplified by the following formula:

(NNS)_{X}

N is either an equal mixture of the deoxynucleotides A, G, C and T, or a mixture consisting of equal amounts of A, T, C, and an excess of G, about 30% more than A. S is an equal mixture of C and G. X is the number of amino acid residues that make up the random peptide sequence, and will vary depending on the characteristics of the random peptide that is sought to be identified. Preferably X is greater than 6 but less than 16. However, it will be appreciated that while this is the preferred size of the random peptide, larger peptides are clearly intended to come within the scope of the invention, as described in detail below.

If the random peptide is constructed as part of a fusion protein, a preferred construct that contains the synthetic DNA degenerate coding sequence that encodes the random peptide may be exemplified by the following formula:

V-----(NNS)ₓ

V stands for nucleotide sequences that encode amino acid (s) found at the amino terminus of a mature protein to which the degenerate coding sequence is fused; N is either an equal mixture of the deoxynucleotides A, G, C and T, or an equal mixture of A, T, C, and an excess of G, about 30%. S is an equal mixture of C and G. X is the number of amino acid residues that make up the random peptide sequence, and will vary depending on the characteristics of the random peptide that is sought to be identified. Preferably X is greater that 6, but less than 16.

It is important to note, and it will be apparent to those skilled in this art, that the size of the random peptide sequence, X, will vary depending on many parameters. A key exemplary parameter is the nature of the protein that is the fusion target. If such proteins have biological activities that contribute to the formation of the random peptide library, then determinative of the size of the random peptide insert will be whether it has deleterious biological effects. For example, it is known that the outer membrane protein, LamB, of E. coli can be altered by genetic insertion to produce hybrid proteins having inserts up to about 60 amino acid residues. Charbit, A., Molla A., Saurin, W., and Hofnung, 1988, Gene, 70:181. Above 60 residues LamB loses significant biological activity.

A more preferred embodiment random peptide cassette construct may be expressed as part of a fusion protein that has the following formula:

V-----(NNS)_{X}----L---V₁

V, and (NNS)_{X}, are as defined above, L stands for an amino acid spacer sequence that facilitates preservation of the active conformation of the random peptide, and serves to present the random peptide sequence to screening reagents. V¹ is the coding sequence of the protein to which the construct is fused.

The synthetic DNA degenerate coding sequence is prepared using known oligonucleotide synthesis techniques. For instance, synthetic oligonucleotides may be prepared by the triester method of Matteucci et al., 1981, J. Am Chem. Soc., 103:3185 or using commercially available automated oligonucleotide synthesizers.

It is worth noting that in a preferred embodiment of the invention, the constructs described above could be designed to encode a cysteine which would facilitate conjugating the peptide to an appropriate substrate. This in turn would facilitate assaying the biological properties of the peptides in those assay formats where it is advantageous to affix the peptide to a solid surface.

A variety of vectors may be used to clone and express the random peptide sequence. Viral or plasmid vectors are preferred. Synthetic DNA that encodes the random peptides is most preferably cloned into viral vectors, and more preferably into bacteriophage vectors. Should the random peptide be expressed as a fusion protein, the preferred viral vectors are the λ gt series, preferably gt11 or derivatives thereof including λ gt11 Sfi-Not (Promega), and filamentous bacteriophage vectors, preferably M13, fl and fd. Most preferred is M13 or derivatives thereof.

A random peptide expressed as part of a fusion protein in λ gt11 is preferably realized by cloning the synthetic DNA into restriction sites at the region of the gene encoding the carboxyl terminal end of an appropriate target protein such as β-galactosidase. The resulting fusion proteins are conveniently assayed using standard λ gt11 screening assays, preferably the methods as described by Davis, R. W., and Young, R. A., in U. S. Patent No. 4,788,135.

A favored embodiment fusion protein construct consists of filamentous bacteriophage in which the random peptide sequences are cloned into phage surface proteins. This has the advantage of screening the random peptide sequence as part of the phage, which is particularly advantageous since the phage may be applied to affinity matrices at over 10¹³ phage /ml, and thus screened in large numbers. Secondly, since a particular phage population may be removed from an affinity matrix and maintain substantial infectious activity, the random peptide may be amplified by subsequent infection of a suitable bacterial host.

For several reasons the preferred phage surface protein is the minor coat protein encoded by gene III of filamentous phage. For instance, foreign antigen epitopes can be expressed in the middle of gene III without significantly disrupting the infectious properties of the phage. Parmley and Smith, 1988, Gene, 73:305. Moreover, five copies of the gene III protein per virion are expressed in E. coli, thus rendering multiple copies available for detection in the screening assays described below.

A preferred embodiment vector of the instant invention that was used to construct the random peptide library is a derivative of M13mp19, termed M13LP67. The construction of this vector, as well as cloning the synthetic degenerate oligonucleotides that encode the random peptide, was carried out using commonly employed techniques known to the skilled practitioner of molecular biology. The reader is particularly referred to Maniatis et al., Molecular Cloning: A Laboratory Manual, Cold Spring Harbor Laboratory, Cold Spring Laboratory, New York, (1982, and 1989, volumes 1 and 2). In addition, many of the materials and methods described herein are also exemplified in Methods & Enzymology, 153-155, Editor Ray Wu/Lawrence Grossman, Academic Press, Inc,. Volume 153 covers methods related to vectors for cloning DNA and for the expression of cloned genes. Particularly note worthy is volume 154, which describes methods for cloning cDNA, identification of various cloned genes and mapping techniques useful to characterize the genes, chemical synthesis and analysis of oligodeoxynucleotides, mutagenesis, and protein engineering. Finally, volume 155 presents the description of restriction enzymes, particularly those discovered in recent years, as well as methods for DNA sequence analysis. These references are hereby incorporated in their entirety, as well as are additional references described below. A general description of the salient methods and materials used is presented here for the convenience of the reader.

More specifically, construction of suitable vectors containing the desired random coding sequence employs standard ligation and restriction methods wherein isolated vectors, DNA sequences, or synthesized oligonucleotides are cleaved, tailored, and religated in the form desired.

Site specific DNA cleavage is performed by treating with suitable restriction enzyme(s) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution. In the examples herein, typically, an excess of restriction enzyme is used to insure complete digestion of the DNA substrate. Incubation times of about 1 to 2 hours at about 37°C are workable, although variations can be tolerated. After each incubation, protein is removed by extraction with phenol/chloroform, and may be followed by ether extraction, and the nucleic acid recovered form aqueous fractions by prccipitation with ethanol followed by chromatography using a Sephadex G-50 spin column. If desired, size separation of the cleaved fragments may be performed by polyacrylamide gel or agarose gel electrophoresis using standard techniques. A general description of size separations is found in Methods in Enzymology, 1980, 65:499-560.

Restriction cleaved fragments may be blunt ended by treating with the large fragment of E. coli DNA polymerase I, that is, the Klenow fragment, in the presence of the four deoxynucleotide triphosphates (dNTPs) using incubation times of about 15 to 25 minutes at 20-25°C in 50 mM Tris pH 7.6, 50 mM NaCl, 6 mM MgCl₂, 6 mM DTT and 10 mM dNTPs. After treatment with Klenow, the mixture is extracted with phenol/chloroform and ethanol precipitated Treatment under appropriate conditions with S1 nuclease results in hydrolysis of single-stranded portions.

Ligations are performed in 15-30 µl volumes under the following standard conditions and temperatures: 20 mM Tris-Cl pH 7.5, 10 mM MgCl₂, 10 mM DTT, 33 µg/ml BSA, 10 mM-50 mM NaCl, and 1 mM ATP, 0.3-0.6 (Weiss) units T4 DNA ligase at 14°C for "sticky end" ligation, or for "blunt end" ligations 1 mM ATP was used, and 0.3-0.6 (Weiss) units T4 ligase. Intermolecular "sticky end" ligations are usually performed at 33-100 µg/ml total DNA concentration. In blunt end ligations, the total DNA concentration of the ends is about 1 µM.

The vector construction employing "vector fragments", the vector fragment is commonly treated with bacterial alkaline phosphatase (BAP) in order to remove the 5' phosphate and prevent religation of the vector. BAP digestions are conducted at pH 8 in approximately 150 mM Tris, in the presence of Na⁺ and Mg⁺² using about 1 unit of BAP per µg of vector at 60°C for about 1 hour. Nucleic acid fragments are recovered by extracting the preparation with phenol/chloroform, followed by ethanol precipitation. Alternatively, religation can be prevented in vectors which have been double digested by additional restriction enzyme digestion of the unwanted fragments.

For portions of the vector which have particular sequence modifications to introduce a desired restriction site, site-specific primer directed mutagenesis was used. For example, it was desirable to modify M13mp19 to introduce restriction sites to produce M13LP67. Site-specific primer directed mutagenesis is now standard in the art, and is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The plaques are transferred to nitrocellulose filters and the "lifts" hybridized with kinased synthetic primer at a temperature which permits hybridization of an exact match, but at which the mismatches with the original strand are sufficient to prevent hybridization. Plaques which hybridize with the probe are then picked and cultured, and the DNA is recovered.

Details of site specific mutation procedures are described below in specific examples. However, site specific mutagenesis can be carried out using any number of procedures known in the art. These techniques are described by Smith, 1985, Annual Review of Genetics, 19:423, and modifications of some of the techniques are described in Methods in Enzymology, 154, part E, (eds.) Wu and Grossman (1987), chapters 17, 18, 19, and 20. A preferred procedure is a modification of the Gapped Duplex site-directed mutagenesis method. The general procedure is described by Kramer, et al., in chapter 17 of the Methods in Enzymology, above.

M13 may be propagated as either a virus, or a plasmid; and if propagated as a plasmid, to facilitate identification of cells that harbor the plasmid, it is desirous that it carry a suitable marker gene. A representative marker gene is β-lactamase, and may be obtained from the plasmids pUC19 or pAc5 by polymerase chain reaction (PCR) amplification. pAc5 is described in WO 89/01029. It was amplified using appropriate primers and standard methods known in the art, or disclosed in U.S. Patent Nos. 4,683,195 issued July 28, 1987; 4,683,202 issued July 28, 1987; and 4,800,159 issued January 24, 1989 the latter of which is incorporated herein by reference in its entirety. A modification of this procedure involving the use of the heat stable Thermus aquaticus (Taq) DNA polymerase has been described and characterized in European Patent Publication No. 258017 published March 2, 1988 incorporated herein by reference in its entirety. PCR is conveniently carried out using the Thermal Cycler instrument (Perkin-Elmer-Cetus) which has been described in European Patent Publication No. 236,069, published September 9, 1987 also incorporated herein by reference in its entirety.

In the constructions set forth below, correct ligations are confirmed by first transforming the appropriate E., coli strain with the ligation mixture. Successful transformants are selected by resistance to ampicillin, tetracycline or other antibiotics, or using other markers depending on the mode of plasmid construction, as is understood in the art. Miniprep DNA can be prepared from the transformants by the method of D. Ish-Howowicz et al., 1981, Nucleic Acids Res., 9:2989 and analyzed by restriction and/or sequenced by the dideoxy method of F. Sanger et al., 1977, Proc. Natl. Acad. Sci. (USA) 74:5463 as further described by Messing et al., 1981, Nucleic Acids Res., 9:309, or by the method of Maxam et al., 1980, Methods in Enzymology, 65:499.

Host strains used to propagate M13 and derivatives include E. coli strains susceptible to phage infection, such as E. coli K12 strain DG98, and strain H249, which is a recA, sup^{o}. F', kan^{r} derivative of MM294. H249 cells are favored if the cells are to be electroporated. The DG98 strain has been deposited with ATCC July 13, 1984 and has Accession No. 1965.

Depending on the host cell used, transformation is done using standard techniques appropriate to such cells. The preferred method is electroporation in a low conductivity solution as described by Dower, W.J., et al., 1988, Nuc. Acids Res., 16:6127. Commercially available electroporation machines may be utilized, such as, for example those made by BTX. Other methods, however, may also be used. For example, the calcium treatment employing calcium chloride, as described by Cohen, S.N., et al., 1972, Proc. Natl. Acad. Sci. (USA) 69:2110, and modifications as described by Hanahan, D., 1983, J. Mol. Biol., 166:557-580 are used for procaryotes or other cells which contain substantial cell wall barriers. Several transfection techniques are available for mammalian cells without such cell walls. The calcium phosphate precipitation method of Graham and Van Der Eb, 1978, Virology 52:546 is one method. Transfection can be carried out using a modification (Wang et al., 1985, Science 228:149) of the calcium phosphate coprecipitation technique. Another transfection technique involves the use of DEAE-dextran (Sompayrac, L.M. et al., 1981, Proc. Natl. Acad. Sci. USA 78:7575-7578). Alternatively, Lipofection refers to a transfection method which uses a lipid matrix to transport plasmid DNA into the host cell. The lipid matrix referred to as Lipofectin Reagent is available from BRL. Lipofectin Reagent comprises an aqueous solution (deionized and sterile filtered water) containing 1 mg/ml of lipid (DOTMA:DOPE, 50:50). This liposome-mediated transfection is carried out essentially as described by Felgner, P.L., et al., 1987, Proc. Natl. Acad. Sci. U.S.A., 84:7413. Lipofectin Reagent and DNA are separately diluted into serum free media so as to avoid gross aggregation which can occur when either material is too concentrated For example, 0.5 X 106 cells are seeded onto a 60 mm tissue culture dish, and 1.5 ml of serum free media containing 1 to 20 µg of DNA and a second solution of 1.5 ml serum free media containing about 30 µg of Lipofectin are prepared. The diluted DNA and Lipofectin solutions are mixed and applied onto the cells. Since transfection is inhibited by serum, the cells are washed well with serum free media before adding the Lipofectin/DNA mixture.

Depending on the properties of the particular random peptide sought, it may be assayed using one or more assay methods. Generally, those methods aimed at detecting the random peptide will detect the random peptide by its capacity to bind an appropriate binding molecule. That is, a molecule that is sought to be tested for random peptide binding activity. Depending on the assay format, it may be labelled or unlabelled. Further, if it is desirable to do so, the random peptide sequence may be purified prior to being employed in the assay.

If the random sequence is part of a fusion protein, preferably a filamentous viral surface protein, the presence of the random peptide sequence may be indicated by the binding of virus to a chosen binding molecule, and separating bound and unbound virus. In this way, virus that contains the random peptide of interest may be isolated, and subsequently amplified by infection of a suitable host cell. Confirmation that the virus encodes a random sequence, as well as the predicted amino acid sequence, can be obtained using standard techniques, including the polymerase chain reaction, and DNA sequencing, respectively.

A random peptide sequence expressing virus may also be revealed using a labelled binding molecule. As applied to viral fusion proteins, preferably lambda bacteriophage, one method is similar to a method described by Davis, R. W., and Young, R. A., in U. S. Patent No. 4,788,135, wherein replica plated phage plaques are contacted with a labelled binding molecule. Fusion proteins produced by phage that express the random peptide sequence will bind the labelled binding molecule, and the phage encoding them can then be identified, isolated and grown up from the replica plates.

Each of the above purification techniques may be repeated multiple times to enrich for the virus that encodes the random peptide of interest.

The binding molecule can be labelled with any type of label that allows for the detection of the binding molecule under the conditions employed, and preferably when the random peptide sequence is bound to a support matrix. Generally, the label directly or indirectly results in a signal which is measurable and related to the amount of random peptide present in the sample. For example, directly measurable labels can include radio-labels (e.g. ¹²⁵I, ³⁵S, ¹⁴C, etc.). A preferred directly measurable label is an enzyme, conjugated to the binding molecule, which produces a color reaction in the presence of an appropriate substrate (e.g. horseradish peroxidase/o-phenylenediamine). An example of an indirectly measurable label is a binding molecule that has been biotinylated. The presence of this label is measured by contacting it with a solution containing a labelled avidin complex, whereby the avidin becomes bound to the biotinylated binding molecules. The label associated with the avidin is then measured. A preferred example of an indirect label is the avidin/biotin system employing an enzyme conjugated to avidin, the enzyme producing a color reaction as described above. Other methods of detection are also usable. For example, avidin could be detected using an appropriately labelled avidin binding antibody.

The following examples are illustrative of various ways in which the invention may be practiced. However, it will be understood by those skilled in the art that the presentation of such examples, showing specific materials and methods, should not be construed as limiting the invention to what is shown in the examples, it being well known by the skilled practitioner that there are numerous substitutions that would perform similarly.

### Example I

### Degenerate Oligonucleotides Encoding Random Peptides

A degenerate oligonucleotide having the following structure was synthesized, and purified using methods known in the art:

5' CTTTCTATTCTCACTCCGCTGAA(NNS)15CCGCCTCCACCTCCACC 3'
5'GGCCGGTGGAGGTGGAGGCGG(XXX)15TTCAGCGGAGTGAGAAT
AGAAAGGTAC 3'

During the synthesis of (NNS)₁₅ mixture consisting of equal amounts of the deoxynucleotides A, C and T, and about 30% more G was used for N, and an equal mixture of C and G for S. X stands for deoxyinosine, and was used because of its capacity to base pair with each of the four bases A,G,C,and T. Reidhaar-Olson, J.F., and Sauer, R. T., 1988, Science, 24:53. Alternatively, other base analogs may be used as described by Habener, J.,et al., 1988, PNAS, 85:1735.

Immediately preceding the nucleotide sequence that encodes the random peptide sequence is a nucleotide sequence that encodes alanine and glutamic residues. These amino acids were included because they correspond to the first two amino terminal residues of the wild type mature gene III protein of M13, and thus may facilitate producing the fusion protein produced as described below.

Immediately following the random peptide sequence, is a nucleotide sequence that encodes 6 prolines residues. Thus, the oligonucleotide encodes the following amino acid sequence:

H₂N-Ala-Glu-Zzz₁₅-Pro₆

Zzz denotes amino acids encoded by the degenerate DNA sequence. As will be described below, the oligonucleotides were cloned into a derivative of M13 to produce a mature fusion protein having the above amino acid sequence, and additionally, following the proline residues the entire wild type mature gene m.

### Example II

### Construction of the Plasmid M13LP67

The plasmid M13LP67 was used to express the random peptide/ gene III fusion protein construct. M13LP67 was derived from M13 mp19 as shown in Figures 1 and 2.

Briefly, M13mp19 was altered in two ways. The first alteration consisted of inserting the marker gene, β-lactamase, into the polylinker region of the virion. This consisted of obtaining the gene by PCR amplification from the plasmid pAc5. The oligonucleotide primers that were annealed to the pAc5 template have the following sequence:

5' GCT GCC CGA GAG ATC TGT ATA TAT GAG TAA ACT TGG 3'
5' GCA GGC TCG GGA ATT CGG GAA ATG TGC GCG GAA CCC 3'

Amplified copies of the β-lactamase gene were digested with the restriction enzymes BglII and EcoRI, and the replicative form of the modified M13mp19 was digested with Bam HI and EcoRI. The desired fragments were purified by gel electrophoresis, ligated, and transformed into E. coli strain DH5 alpha (BRL). E. coli transformed with phage that carried the insert were selected on ampicillin plates. The phage so produced were termed JD32.

The plasmid form of the phage, pJD32 (M13mp19Ampr), was mutagenized so that two restriction sites, EagI and KpnI, were introduced into gene III without altering the amino acids encoded in this region. The restriction sites were introduced using standard PCR in vitro mutagenesis techniques as described by Innis, M., et al. in PCR Protocols-A Guide to Methods and Applications (1990), Academic Press, Inc.

The KpnI site was constructed by converting the sequence, TGTTCC, at position 1611 to GGTACC. The two oligonucleotides used to effect the mutagenesis have the following sequence:

LP159:	AAACTTCCTCATGAAAAAGTC
LP162:	AGAATAGAAAGGTACCACTAAAGGA

To construct the EagI restriction site, the sequence at position 1631 of pJD32, CCGCTG, was changed to CGGCCG using the following two oligonucleotides:

LP160:	TTTAGTGGTACCTTTCTATTCTCACTCGGCCGAAACTGT
LP161:	AAAGCGCAGTCTCTGAATTTACCG

More specifically, the PCR products obtained using the primers LP 159, LP 162 and LP 160 and LP161 were digested with BspHI and KpnI, and KpnI and AlwNI, respectively. These were ligated with T4 ligase to M13mp19 previously cut with BSpHI and AlwNI to yield M13mpLP66. This vector contains the desired EagI and KpnI restriction sites, but lacks the ampicillin resistance gene, β-lactamase. Thus, the vector M13mpLP67, which contains the EagI and KpnI restriction sites and β-lactamase was produced by removing the β-lactamase sequences from pJD32 by digesting the vector with XbaI and EcoRI. The β-lactamase gene was then inserted into the polylinker region of M13mpLP66 which was previously digested with XbaI and EcoRI. Subsequent ligation with T4 ligase produced M13mpLP67, which was used to generate the random peptide library. Figures 1 and 2 schematically sets forth the construction of M13mpLP67.

### Example III

### Production of Phage Encoding Random Peptides

To produce phage having DNA sequences that encode random peptide sequence, M13LP67 was digested with EagI and KpnI, and ligated to the oligonucleotides produced as described, in Example I, above. The ligation mixture consisted of digested M13LP67 DNA at 45 ng/µl, a 5-fold molar excess of oligonucleotides, 3.6 U/µl of T4 ligase (New England Biolabs), 25 mM Tris, pH 7.8, 10 mM MgCl₂, 2 mM DTT, 0.4 mM ATP, and 0.1 mg/ml BSA. Prior to being added to the ligation mixture, the individual oligonucleotides were combined and heated to 95°C for 5 minutes, and subsequently cooled to room temperature in 15 µl aliquotes. Next, the ligation mixture was incubated for 4 hours at room temperature and subsequently overnight at 15°C. This mixture was then electroporated into E. coli as described below.

M13LP67 DNA was electroporated into H249 cells that were prepared essentially as described by Dower, W., J. J. Miller, J. F. and Ragsdale, C. W., 1988, Nucleic Acids Research. 16:6127. H249 cells are a recA, supo, F' kan^{R} derivative of MM294. Briefly, 4 x 10⁹ H249 cells and 1 µg of M13LP67 DNA were combined in 85 ul of a low conductivity solution consisting of 1 mM Hepes. The cell/M13LP67DNA mixture was positioned in a chilled 0.56 mm gap electrode of a BTX electroporation device (BTX Corp.) and subjected to a 5 millisecond pulse of 560 volts.

Immediately following electroporation, the cells were removed from the electrode assembly, mixed with fresh H249 lawn cells, and plated at a density of about 2 x 10⁵ plaques per 400 cm² plate. The next day phage from each plate were eluted with 30 ml of fresh media, PEG precipitated, resuspended in 20% glycerol, and stored frozen at -70°C. About 2.8 x 10⁷ plaques were harvested and several hundred analyzed to determine the approximate number that harbor random peptide sequences. Using the polymerase chain reaction to amplify DNA in the region that encodes the random peptide sequence, it was determined that about 50-90 % of the phage contained a 69 base pair insert at the 5' end of gene III. This confirmed the presence of the oligonucleotides that encode the random peptides sequences. The PCR reaction was conducted using standard techniques and with the following oligonucleotides: 
5' TCG AAA GCA AGC TGA TAA ACC G 3'
5' ACA GAC AGC CCT CAT AGT TAG CG 3'
 The reaction was run for 40 cycles after which the products were resolved by electrophoresis in a 2% agarose gel. Based on these results, it was calculated that phage from the 2.8 x 10⁷ plaques encode about 2 x 10⁷ different random amino acid sequences.

### Example IV

### Properties of Phage Encoded Random Peptides

The random peptide library was screened for peptide binding activity to streptavidin as follows. Streptavidin was bound to a solid matrix and used to screen for phage that carry streptavidin binding peptides on their surface using biopanning techniques well known in the art. This procedure was essentially conducted as described by Parmley S. F. and Smith, G. P., 1988, Gene, 73:305.

Briefly, 60 mm polystyrene plates (60 x 15 mm, Falcon Corp.) were coated overnight with lmg/ml of streptavidin in 0.1 M NaHCO₃, pH 8.6, containing 0.02% Na N³ to prevent bacterial growth. The next day the streptavidin solution was removed, and the plates were blocked for at least one hour with 10 ml of a blocking solution consisting of 29 mg/ml of bovine serum albumin, 3 µg/ml streptavidin, 0.02 % NaN_{3,} in 0.1 M NaHCO₃, pH 8.6. Next, the plates were rinsed with TBS-Tween and 10¹² phage adsorbed onto the plates for 15 minutes, followed by washing the plates ten times with TBS-Tween to remove phage that did not bind to streptavidin. TBS-Tween consisted of 50 mM Tris-HCl, pH 7.5, 150 mM NaCl, and 0.5% Tween 20. The adherent phage were eluted from the plates with 800 µl of a sterile solution consisting of 6 M Urea in 0.1 N HCl. (pH adjusted to 2.2 with glycine) The phage were eluted in this solution for 15 minutes, and the solution neutralized with 23 µl of 2 M Tris Base. This procedure yielded about 4 x 10⁵ phage. A stock of the phage was prepared by reinfection of E. coli and preparation of a plate stock, using standard procedures, and the streptavidin biopanning selection procedure repeated. In the second selection, 10¹⁰ phage were panned, and 10⁸ were finally eluted. These phage were plated at low density, and 60 separate phage stocks were prepared from randomly selected, individual plaques.

The streptavidin binding properties of the 60 isolates were studied in detail. To insure that the phage did indeed display a peptide sequence with streptavidin binding activity, an experiment was conducted to determine if the isolates could be enriched from an excess of phage that did not carry random peptide inserts. Thus, each of the isolates was mixed with M13mp19, and the mixtures were panned on streptavidin plates, and adherent virus eluted as described above. The ratio of random peptide encoding phage to M13mp19 in the initial mixture before biopanning, and the ratio in the eluate was compared by plating the two phage populations on Xgal plates. The two populations could be distinguished because M13mp19 phage form blue plaques, while M13LP67 random peptide sequence expressing phage form white plaques. It was observed that 56 of the 60 isolates were enriched at least 10 fold over M13mp19. A control was run in which both populations of phage were biopanned, as described above, with the exception that the polystyrene dishes were not treated with streptavidin. No enrichment for the random peptide expressing phage was observed. Further, Table 1 shows that 9 of the 60 isolates are enriched from 8 x 10³ to 6.7 x 10⁴ fold in the presence of M13mp19. The Table also shows that 2 of the isolates failed to bind to streptavidin.

**Table 1**

| | - Biotin | | | + Biotin | | |
|---|---|---|---|---|---|---|
| | Number of M13mp19 Plaques Per Isolate Plaque | | | Number of M13mp19 Plaques Per Isolate Plaque | | |
| Isolate | Initial | Eluate | Enrichment | Initial | Eluate | Enrichment |
| A | 28 | 0.002 | 1.4 x 10⁴ | | | |
| B | 58 | 0.003 | 1.9 x 10⁴ | 25 | 2 | 13 |
| C | 19 | 0.0007 | 2.6 x 10⁴ | | | |
| D | 60 | 0.0009 | 6.7 x 10⁴ | 320 | 3 | 107 |
| E | 140 | 0.012 | 1.2 x 10⁴ | 110 | 1 | 110 |
| F | 23 | 0.0091 | 2.5 x 10³ | | | |
| G | 28 | 0.0009 | 3.1 x 10⁴ | 21 | 2 | 10 |
| H | 26 | 0.0024 | 1.1 x 10⁴ | 48 | 1 | 48 |
| I | 16 | 0.002 | 8.0 x 10³ | 17 | 6 | 2.8 |
| | | | | | | |
| Y | 11 | 5 | 2.2 | | | |
| Z | 15 | 4 | 3.8 | | | |
| M13LP67 | 9 | 5 | 1.8 | | | |

To further characterize the streptavidin binding properties associated with certain of the isolates, the biopanning experiment with M13mp19 was repeated but with the addition of 1 µM biotin to the panning solution. Biotin binds very tightly to streptavidin, and may be expected to reduce the binding activity of the selected phage to streptavidin. Indeed, Table 1 shows a large reduction in enrichment for random peptide expressing phage when biotin is present.

The DNA that encodes the random peptide sequences from the isolates A through I, shown in Table 1, was sequenced, and the predicted amino acid sequences of the random sequences is shown in Table 2. It is apparent that they exhibit a histidine-proline consequence sequence. The random peptide inserts of 6 isolates that did not exhibit streptavidin binding were also sequenced, and they did not display the histidine-proline consensus sequence.

Table 2 presents the predicted amino acid sequence of the random peptides. encoded by several streptavidin binding phage.

**Table 2**

| Isolate | Frequency | | | |
|---|---|---|---|---|
| A | 3 | SDDWWHD | HPQN | LRSS |
| B | 1 | MLWYSPHSFS | HPQN | T |
| C | 1 | SWWLSW | HPQN | TKELG |
| D | 5 | ISFENTWLW | HPQF | SS |
| E | 1 | LC | HPQF | PRCNLFRKV |
| F | 2 | PC | HPQY | RLCQRPLKQ |
| G | 2 | QPFL | HPQG | DERWYMI |
| H | 1 | ALCCLSSP | HPNG | AIF |
| I | 4 | LN | HPMD | NRLHVSTSP |
| | | | | |
| Consensus | | | HP | |

To confirm that the consensus sequence, histidine-proline, is indeed responsible for binding to streptavidin, a peptide was synthesized that has the consensus sequence and tested for inhibition of streptavidin binding as described above. The peptide was observed to significantly inhibit streptavidin binding. The peptide was synthesized using known methods, and had the following sequence:

Leu-Asn-His-Pro-Met-Asp-Asn-Arg-Leu-His-Gly-COOH

### Example V

### Construction of Random Peptide Library in λ Phage

Ten µg of λ gt 11 Sfi-Not was cut with EcoRI and Not 1, phenol/chloroform extracted, ethanol precipitated, washed in 80% ethanol, and resuspended in 10 µl TE buffer. We then mixed 12.5 picomoles of each of the following two oligonucleotides in 2 µl of water.

5'-GGCCGCTCAATCAGTCAXXXXXXXXXXXXXXXXXXXXXXXXXXXX
XXXXXXXXXXXXXXXXXGGGCGGCGGAGGTGGCGG-3'
5'-AATTCTCCGCCACCTCCGCCGCCCNNSNNSNNSNNSNNSNNSNNSNN
SNNSNNSNNSNNSNNSNNSNNSTGACTGATTGACG-3'

Next, the oligonucleotides were heated to 80°C for 2 minutes cooled, and mixed with an additional 10 µl of H₂O, 2 µl of 10 x Ligase mix (described by Maniatis, above), 5 µl of the cut DNA described above and 1 µl of T4 DNA ligase (England Biolabs, 100,000 - 500,000 U/ml) and incubated at room temperature for 3 hours, and subsequently at 15°C for 2.5 days. Sixty percent of the mixture was then loaded onto a 0.5% agarose TEA gel in one 5 x 5 x 1.5 mm well. After electrophoresis, the high molecular weight DNA band was excised from the gel, and placed in a solution consisting of 100 mM NaCl, 30 mM Tris, pH 8 for 1 to 12 hours. The gel fragment was then removed from the blotted with a kim wipe to remove excess solution and melted at 70°C and 5 µl of the molten agarose was placed in each of 2 tubes. The tubes were heated to 90°C for 2 minutes and 6 minutes, respectively, and incubated at 65°C for 2 hours. 2.5 µl from each tube was packaged using Stratagene Corporation's Giga pack plus phage and packing kit. These packaged phage were used to infect Y1090 cells (Promega) and plated at a density of 50,000 plaques per 150 mm diameter plate. 4 x 10⁶ independent plaques were generated by this method and plate stocks were prepared.

The above λ phage library was screened with a monoclonal antibody to feline leukemia virus that recognizes the peptide QAMGPNLVL. The antibody is described by Nunberg, J. M. et al., 1984, PNAS, 81:3675, while the peptide is described by Kuldip, S. et al., 1989, in Peptides--Chemistry, Structure and Biology, (eds., Jean Rivier and Garland Marshall) Escom, Leiden , 1990. Standard λ gt11 screening techniques were used, as described by Davis, R. W., and Young, R. A., in U. S. Patent No. 4,788,135. More than 6 different phage were isolated. The antibody bound to protein on nitrocellulose filters that had been overlaid on plaques created by these phage, thus indicating the presence of a peptide sequence in the phage. This binding could be greatly reduced by co-incubating the filters and antibodies with 25 µg/ml of the peptide QAMGPNLVL, but an irrelevant peptide had no effect.

The present invention has been described with reference to specific embodiments. However, this application is intended to cover those changes and substitutions which may be made by those skilled in the art without departing from the spirit and the scope of the appended claims.

## Claims

1. A random peptide library comprising a plurality of fusion proteins, each fusion protein comprising a viral surface protein and a random peptide sequence of between six and sixteen amino acids at the amino terminal region of the viral surface protein wherein each fusion protein has a linker of about six amino acids between the C-terminus of the said peptide sequence and the viral surface protein.

2. A random peptide library according to claim 1, wherein said each said peptide sequence comprises about fifteen amino acid residues.

3. A random peptide library according to claim 1 or 2, wherein each said linker comprises proline.

4. A random peptide library according to claim 3, wherein each said linker comprises about six prolines.

5. A random peptide library according to any one of the preceding claims wherein the viral surface protein is a filamentous phage surface protein.

6. A random peptide library according to claim 5 in a filamentous phage expression system.

7. A library of nucleic acid sequences encoding a random peptide library according to any one of the preceding claims.

8. A cell transformed with the mixture of nucleic acid sequences according to claim 7.

9. A cell according to claim 8 wherein the cell is a bacterium.

10. A method for producing a random peptide library according to any one of claims 1 to 5 comprising expressing nucleic acid sequences encoding a plurality of fusion proteins in a cell, each fusion protein comprising a viral surface protein and a peptide sequence of between six and sixteen amino acids at the amino terminal region of the viral surface protein wherein each fusion protein has a linker of about six amino acids between the C-terminus of the said peptide sequence and the viral surface protein and optionally purifying the random peptide library.

11. Use of a random peptide library according to any one of claims 1 to 6 for identifying and selecting peptides that have a particular biological activity or to isolate and purify ligand binding molecules.

## Patentansprüche

1. Zufallspeptidbank, umfassend eine Vielzahl von Fusionsproteinen, wobei jedes Fusionsprotein ein virales Oberflächenprotein und eine Zufallspeptidsequenz von 6 bis 16 Aminosäuren am aminoterminalen Ende des viralen Oberflächenproteins umfaßt, wobei jedes Fusionsprotein einen Linker von etwa 6 Aminosäuren zwischen dem C-Terminus der Peptidsequenz und dem viralen Oberflächenprotein aufweist.

2. Zufallspeptidbank nach Anspruch 1, wobei jede Peptidsequenz etwa 15 Aminosäurereste umfaßt.

3. Zufallspeptidbank nach Anspruch 1 oder 2, wobei jeder Linker Prolin umfaßt.

4. Zufallspeptidbank nach Anspruch 3, wobei jeder Linker etwa 6 Proline umfaßt.

5. Zufallspeptidbank nach einem der vorangehenden Ansprüche, wobei das virale Oberflächenprotein ein Oberflächenprotein eines filamentösen Phagen ist.

6. Zufallspeptidbank nach Anspruch 5 in einem Expressionssystem eines filamentösen Phagen.

7. Bank von Nucleinsäuresequenzen, die eine Zufallspeptidbank nach einem der vorangehenden Ansprüche codieren.

8. Zelle, die mit dem Gemisch von Nucleinsäuresequenzen nach Anspruch 7 transformiert ist.

9. Zelle nach Anspruch 8, wobei die Zelle ein Bakterium ist.

10. Verfahren zur Herstellung einer Zufallspeptidbank nach einem der Anspruche 1 bis 5, umfassend die Expression von Nucleinsäuresequenzen, die eine Vielzahl von Fusionsproteinen in einer Zelle codieren, wobei jedes Fusionsprotein ein virales Oberflächenprotein und eine Peptidsequenz von 6 bis 16 Aminosäuren am aminoterminalen Ende des viralen Oberflächenproteins umfaßt, wobei jedes Fusionsprotein einen Linker von etwa 6 Aminosäuren zwischen dem C-Terminus der Peptidsequenz und dem viralen Oberflächenprotein aufweist, und gegebenenfalls Reinigung der Zufallspeptidbank.

11. Verwendung einer Zufallspeptidbank nach einem der Ansprüche 1 bis 6 zur Identifizierung und Auswahl von Peptiden, die eine bestimmte biologische Aktivität aufweisen, oder zur Isolierung und Reinigung von Liganden-bindenden Molekülen.

## Revendications

1. Banque de peptides aléatoires comprenant une pluralité de protéines de fusion, chaque protéine de fusion comprenant une protéine de surface de virus et une séquence peptidique aléatoire constituée de six à seize acides aminés au niveau de la région amino-terminale de la protéine de surface du virus, où chaque protéine de fusion possède un lieur d'environ six acides aminés entre le site C-terminal de ladite séquence peptidique et la protéine de surface du virus.

2. Banque de peptides aléatoires selon la revendication 1, dans laquelle chacune desdites séquences peptidiques comprend au moins 15 résidus d'acides aminés.

3. Banque de peptides aléatoires selon la revendication 1 ou 2, dans laquelle chaque lieur comprend une proline.

4. Banque de peptides aléatoires selon la revendication 3, dans laquelle chaque lieur comprend environ six prolines.

5. Banque de peptides aléatoires selon l'une quelconque des revendications précédentes, dans laquelle la protéine de surface du virus est une protéine de surface de phage filamentaire.

6. Banque de peptides aléatoires selon la revendication 5, dans un système d'expression de phage filamentaire.

7. Banque de séquences d'acides nucléiques codant une banque de peptides aléatoires selon l'une quelconque des revendications précédentes.

8. Cellule transformée par le mélange de séquences d'acide nucléique selon la revendication 7.

9. Cellule selon la revendication 8, dans laquelle la cellule est une bactérie.

10. Procédé pour produire une banque de peptides aléatoires selon l'une quelconque des revendications 1 à 5, qui consiste à exprimer des séquences d'acide nucléique codant une pluralité de protéines de fusion dans une cellule, chaque protéine de fusion comprenant une surface de protéine de virus et une séquence peptidique constituée de six à seize acides aminés dans la région amino-terminale de la protéine de surface du virus, où chaque protéine de fusion possède un lieur d'environ six acides aminés entre l'extrémité C-terminale de ladite séquence peptidique et la protéine de surface du virus, et éventuellement à purifier la banque de peptides aléatoires.

11. Utilisation d'une banque de peptides aléatoires selon l'une quelconque des revendications 1 à 6, pour identifier et sélectionner des peptides ayant une activité biologique particulière, ou pour isoler et purifier des molécules de liaison à un ligand.
